# EUROPEAN PATENT APPLICATION

(11) **EP 4 631 574 A2**
(43) Date of publication of application: **15.10.2025**
(21) Application number: 25198575.0
(22) Date of filing: 22.03.2021
(51) Int. Cl.: A61P 37/02

(54) **HIGHLY PURIFIED EICOSAPENTAENOIC ACID, AS FREE FATTY ACID FOR TREATMENT OF CORONAVIRUSES AND SPECIFICALLY NOVEL CORONOAVIRUS-19 (COVID-19)**

(30) Priority: 23.03.2020 US 202062993109 P; 14.04.2020 US 202063009575 P; 28.04.2020 US 202063016414 P
(62) Divisional of application: 21721986.4
(71) Applicant: KD Swiss GmbH, 6300 Zug (CH)
(72) Inventor: BELLUZZI, Andrea, 40127 Bologna (IT); LEICESTER, Roger James, New Malden, KT3 5DS (GB)
(74) Representative: Patentanwälte Bernhardt / Wolff Partnerschaft mbB

(57) **Abstract**

This present invention relates to the use of eicosapentaenoic acid (EPA) for treating and reducing the negative effects of respiratory virus including coronaviruses and particularly the novel coronavirus-19 (COVID-19). Notably, the eicosapentaenoic acid in the free fatty acid (EPA-FFA) form has a purity of at least has a purity of at least 90%, preferably 95% and more preferably at least 99%. Specifically, the EPA-FFA exhibits a reduction of expressed interleukins, such as IL-6, reduction in D-dimers and a reduction relating to the release of AA.

## Description

### CROSS-REFERENCE TO RELATED APPLICATIONS

The present application claims priority to U.S. Provisional Patent Application Serial No. 62/993,109, filed on March 23, 2020, U.S. Provisional Patent Application Serial No. 63/009,575, filed on April 14, 2020, and U.S. Provisional Patent Application Serial No. 63/016,414, filed on April 28, 2020, the contents of all are incorporated herein by reference in their entirety.

### BACKGROUND OF THE INVENTION

### Technical Field

This invention relates to the use of highly purified eicosapentaenoic acid as free fatty acids (EPA-FFA) for treating and reducing the negative effects of a respiratory virus, such as coronaviruses and particularly the novel coronavirus-19 (COVID-19) and variants thereof.

### Related Art

The coronaviruses are enveloped viruses, having a capsid exhibiting a helical symmetry. They have a single-stranded positive sense RNA genome and are capable of infecting cells from birds and mammals. The viruses which are members of this very wide family are known to be causative agents for cold (for example hCoV and OC43 viruses), bronchiolitis (for example NL63 virus) or even some forms of several pneumoniae as those observed during the SARS (such as the Severe Acute Respiratory Syndrome Coronavirus, SARS-CoV) epidemic.

Although they belong to a same viral family, significant differences exist between the different coronaviruses, both at the genetic and structural level and are the common etiological agents of mild to moderate upper respiratory tract infections. However, novel coronaviruses such as Middle Eastern Respiratory Syndrome Coronavirus (MERS-CoV) can result in severe lower respiratory tract infections and high mortality. MERS-CoV was first identified in 2012 within the Arabian Peninsula and since its initial outbreak, Sporadic MERS-CoV infections continue to appear within the Arabian Peninsula. The epidemiology of MERS-CoV infection in humans remains unclear and convoluted with bats and Dromedary camels being the major reservoirs for the virus.

In January 2020 the World Health Organization reported infection from a new novel coronavirus COVID-19 which is showing a higher mortality rate than MERS-CO-V or SARS and exhibits an ongoing risk of human to human transmission. To date, there is no prophylactic or therapeutic solution to efficiently treat the negative effects of this epidemic respiratory viral pathogen that has reached a pandemic level. The absence of a treatment to reduce the negative effects of COVID-19 poses a severe global health threat due to its pandemic determination. As such, there is a necessity to provide a new and effective treatment to reduce the negative effects of COVID-19 and variants thereof.

### SUMMARY OF THE INVENTION

The present invention provides the use of highly purified eicosapentaenoic acid as free fatty acids (EPA-FFA) for treating and reducing the effects of a coronavirus and particularly Novel Coronavirus-19 (COVID-19) and variants thereof. Preferably, the eicosapentaenoic acid in the free fatty acid (EPA-FFA) form has a purity of at least has a purity of at least 90%, preferably 95% and more preferably at least 99%. Importantly it has been found that EPA in the free fatty acid form is far more bioavailable than in the ethyl ester (EE) form. Specifically, the EPA-FFA exhibits a therapeutic effect by reducing the overactive immune response, specifically the high expression levels of cytokines. Thus, this EPA-FFA preparation, not only modifies the COVID-19 and variants thereof disease process by reducing harmful excess inflammatory responses, but to do so without suppressing the immune response to the virus which are vital to seroconversion giving the patient ongoing protection against continued viral challenge. As such, EPA-FFA is useful as a therapeutic agent, in particular in the treatment, therapy or prophylaxis of this novel coronavirus .

Accumulating evidence suggests that some patients with severe COVID-19 and variants thereof are experiencing numerous adverse effects including strokes from blood clots, neurological issues, loss of smell and taste (anosmia), unexpected blood clotting, inflamed alveoli (air sacs) which hampers breathing; pulmonary embolism from breakaway blood clots and microclots, weakened heart muscle which causes dangerous arrhythmias and heart attacks due to small clots, damage to kidneys wherein patients often require dialysis and also overactive immune response that attacks healthy tissue which is consider a cytokine storm syndrome. The use of EPA-FFA as a therapeutic agent, in particular in the treatment, therapy or prophylaxis of this novel coronavirus can address these symptoms.

Cytokines are inflammatory immunologic proteins that are in the body to fight off infections but too many can result in an immune system gone wild. The body's own killer immune cells, in such an increased amount, can lead to organ failure and death. Currently, respiratory failure from acute respiratory distress syndrome (ARDS) is the leading cause of mortality of patients with COVID-19 and variants thereof. Thus, the present invention provides for a novel approach to reduce inflammatory cytokine proteins, which can include interleukins IL-1, IL-6 and IL-2 and to treat ill individuals with complications of hyper-inflammation due to COVID-19 and variants thereof.

Further the present invention provides for a composition and method using the virally effective EPA-FFA composition to reduce levels of D-dimers in a treated subject. Some important markers of organ dysfunction and coagulopathy found in subjects with Covid-19 includes higher levels like LDH (Lactate Dehydrogenase) and D-Dimers. D-dimers are protein products of cross-linked fibrin degradation that are present in the blood of most healthy individuals but have been found in dangerous levels in subjects experiencing the effects of COVID-19 and other respiratory viral infections, including viral pneumonia due to influenza. Thus, the reduction of D-Dimers is another advantageous effect of the present invention.

Notably, higher percentage of CD14+ CD16+ inflammatory monocytes exist in peripheral blood of COVID-19 and variants thereof patients. The percentage of CD14+ CD16+ monocytes is much higher in patients exhibiting ARDS. Moreover, a significantly higher expression of IL-6 is secreted from these inflammatory monocytes especially in patients with ARDS. This increase in IL-6 + monocytes can be related to the cytokine storm caused by monocytes that can migrate to the lung area. Thus, in COVID-19 and variants thereof patients these activated immune cells may enter the pulmonary circulation in large quantities and exhibit an immune damaging role in ARDS.

One method to deal with the cytokine storm syndrome is the treatment with corticosteroids which act as broad immunosuppressive agents. However, there can be some concern related to treating a severely ill, infected individual with such powerful and wide-ranging immune suppression. Thus, the present invention provides an alternative by treatment with a therapeutically-effective amount of EPA-FFA.

In one aspect, the present invention also provides a method of treating a coronavirus-infected cell or organism comprising treatment with an effective amount of EPA-FFA having a purity of at least has a purity of at least 90%, preferably 95% and more preferably at least 99% which reaches the interior of the cell or the target site in the organism.

In another aspect, the present invention provides a method for alleviating the cytopathic destructive effects of COVID-19 and variants thereof infection in a human patient comprising administering to said patient a therapeutically-effective amount of EPA-FFA having a purity of at least has a purity of at least 90%, preferably 95% and more preferably at least 99% or a composition containing such a compound.

In yet another aspect, the present invention provides for therapeutic compositions comprising a therapeutically-effective amount of EPA-FFA having a purity of at least has a purity of at least 90%, preferably 95% and more preferably at least 99% in a pharmaceutically-acceptable carrier.

In addition, because the use of more than one active agent may provide a better therapeutic composition, and this is particularly true when the different agents act by different mechanisms, this invention also includes therapeutic compositions comprising both EPA-FFA according to the present invention together with one or more other antiviral agents, such as Reverse transcriptase (RT) inhibitors - interfere with a critical step during the virus life cycle and keep the virus from making copies of itself; Nucleoside analogs - nucleoside analogue to inhibit coronavirus replication due to lethal mutagenesis: Protease inhibitors - interfere with a protein that viruses use to make infectious viral particles; Fusion inhibitors - block the virus from entering the body's cells; Integrase inhibitors - can block an enzyme needed to make copies of itself; Multidrug combinations - combine two or more different types of drugs into one, and/or Pharmacokinetic Enhancers, as well as biological response modifiers, including, for example, interferon (α, β or γ), interleukin-2 and granulocyte-macrophage colony stimulating factor ("GM-CSF") and the like. Also the use of dexamethasone is acceptable and preferably in an amount as an oral (liquid or tablets) or intravenous preparation of about 4 mg to 8 mg daily for a period of 1 to 2 weeks.

Combination compositions of the present invention may comprise lower doses of the active antiviral agent while maintaining a level of antiviral activity that is characteristic of a higher dose. As a result, the cytotoxicity typically associated with the administration of an antiviral agent is minimized by the administration of combination compositions of the present invention. Combination compositions may comprise a reduced dosage of an antiviral agent in combination with the EPA-FFA of the present invention to achieve a level of antiviral activity that is greater than that normally required while maintaining an acceptable level of cytotoxicity.

In a further aspect, the present invention provides for a combination of EPA-FFA, steroids that do not suppress all immune response, such as dexamethasone. DHA and other long chain fatty acids and acetylsalicylic acid or a metabolite thereof.

For purposes of this invention, the terms " therapeutically-effective" amount means an amount of the EPA-FFA compound according to the invention that results in treatment, prophylaxis, slowing of spread of the coronavirus including COVID-19 and variants thereof or of manifestations of coronavirus, prevention of infection of others and/or improvement in patient condition.

In yet another aspect, the present invention provides for therapeutic methods of treating subjects (e.g., vertebrates, such as humans) by modulating an immune response caused by increase in the expression of interleukins and the release of arachidonic acid (AA), the method comprising the administration to a subject a therapeutically effective amount of EPA-FFA to stop the cascade of AA and suppress the activity of NFκB thereby reducing the levels of interleukins and release of AA.

In light of recent understanding of the SARS-Cov2 infection, it is known that besides the acute phase, a frightening later complication can present itself, that being pulmonary fibrosis. It was found that as the disease progressed, the range of ground glass density patches and consolidation increased, and the resolution phase is characterized by little fibrous stripes. In some patients even if only few fibrous stripes are seen at an early stage, consolidation can develop in the short term reexamination. (1)

Thus, in a further aspect the present invention provides for administering EPA-FFA of the present invention to a subject suffering from additional complications from COVID 19 to decreases the production of IL-6 and VEGF (vascular endothelial growth factor) by inhibiting ERK (mitogen-activated protein kinase) and angiogenesis that ultimately lead to collagen production by fibroblast (2). It is believed that EPA FFA reduces the level of inflammatory immune response, by reducing IL-6 and VEGF production and the secondary neo angiogenesis that ultimately generates pulmonary fibrosis. As such, EPA-FFA treatment in a late stage of SARS-Cov2 infection, may reduce one of the late complication events, that being lung fibrosis in those patients

In a still further aspect, the present invention provides for a kit for treating a subject against COVID-19 and variants thereof wherein the kit comprises a therapeutically effective amount of EPA-FFA of the present invention and may further comprise instructions for use.

In yet another aspect, the present invention provides for the use of a therapeutically effective amount of EPA-FFA in a medicament or in the manufacture of a medicament for the treatment of the negative effects due to COVID-19 and variants thereof.

These and other advantages and features of the present invention will be described more fully in a detailed description of the preferred embodiments which follows.

### BRIEF DESCRIPTION OF THE FIGURES

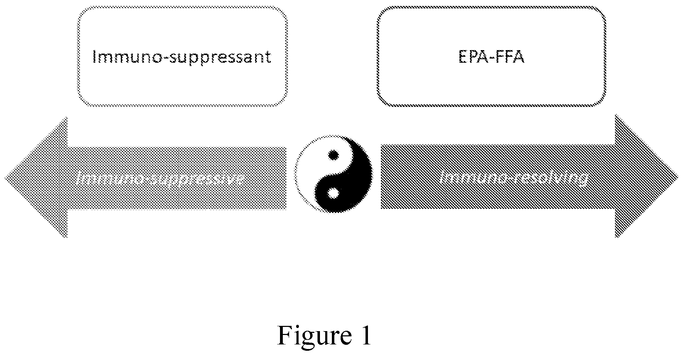

Figure 1 indication that use of the EPA-FFA of the present lacks immune-suppressive activity while retaining an immune-resolving or immune-modulating approach to a respiratory virus, such as COVID -19 infection.

### DETAILED DESCRIPTION OF THE INVENTION

It is known that COVID-19 and variants thereof can stimulate a cytokine storm in the lung, such as an increase of IL-2, IL-6, IL-7, GSCF, IP10, MCP1, MIP1A, and TNFα, followed by the edema, dysfunction of the air exchange, acute respiratory distress syndrome, acute cardiac injury and the secondary infection which may lead to death. Clearly, avoiding the cytokine storm may be the key for the treatment of COVID-19 and variants thereof infected patients. Thus, the administration of EPA-FFA, owing to its powerful immunomodulatory ability, can have beneficial effects for preventing or attenuating the cytokine storm.

The present invention is based on the findings that administration of EPA-FFA is effective *in vivo* in treating and reducing the negative effects of infection by COVID-19 and variants thereof. It is theorized that EPA-FFA influences an overactive immune response and specifically reduces the high levels of cytokines that cause respiratory failure from ARDS which is the leading cause of mortality of patients with COVID-19 and variants thereof.

Variants or variants of the COVID-19 may include changes in the amino acids found in the spike protein of the virus, but also in the ORF protein region. For example, the B.1.1.7 variant has a notable mutation N501Y. Other mutations have been found to have more infective including A222V, E484K, S477N and K417N/T. As the virus spreads and more people are infected additional variants are inevitable.

As a background for understanding the COVID-19 and variants thereof and effects caused by EPA-FFA it is helpful to understand the initiation of an immune response. Toll-like receptors (TLRs) are a family of receptors located on the surface of a cell. TLR4 once stimulated by a lipopolysaccharide, such as during a COVID-19 and variants thereof infection, activates signal transduction through the NFκB pathway. The identification of NFκB signaling pathways as important drivers of human disease and the role of the IKK complex as the ubiquitous signal integration hub for NF-κB activation pathways has led to identifying inhibitors of the IKKs, and specifically inhibitors of the IKKβ kinase. IKKβ kinase is involved in the displacement of NFκB from the cytoplasm towards the nucleus. Once the NFκB reaches the nucleus, it significantly increases the expression of interleukins, such as IL-1, IL-6 and IL-2 and TNF. These stimuli also increase the availability and the release of arachidonic acid (AA). The free released AA is subsequently metabolized by several major enzymatic pathways including the cyclooxygenase (COX) and lipoxygenase (LO) pathways. Of interest, COX-2 is primarily involved in inflammation. Other AA metabolites derived from the 5-LO pathway known as the leukotrienes (LTs) are involved in mediating and maintaining inflammation. Leukotrienes are a family of eicosanoid inflammatory mediators produced by the oxidation of arachidonic acid. Consequently, drugs able to inhibit 5-LO are considered helpful to address the inflammatory cytokines storm of COVID-19 and variants thereof. In China, coronavirus patients exhibiting signs of cytokine-storm syndrome reportedly are being treated with the anti-inflammation drug Actemra (tocilizumab). Actemra is used in the U.S. for rheumatoid arthritis.

The present inventors have found that increase levels of eicosapentaenoic acid in the free fatty acid form exhibits an anti-inflammatory effect. For example, IKKβ is uniquely and exclusively limited physiologically by EPA and by vitamin B. This is crucial because the use of EPA-FFA can act as a biological "break" (suppressor) - which delays or slows down the inflammatory storm. Secondary to that it plays an essential role in the ratio of AA to EPA present in the membranes.

Eating habits have changed dramatically over the years, leading to an imbalance in the ratio of n-6/n-3 polyunsaturated fatty acids (PUFAs) in favor of n-6 PUF As, particularly in the Western diet. Meanwhile, the incidence of inflammatory diseases is increasing worldwide. For example, in Europe there is an estimate of a ratio of 30:1 (AA:EPA), when the norm is 1:1 or at most 5:1. In the United States it is worse, that being 75:1. This ratio is very pro-inflammatory. This explains the effect of AA in the hyper-inflammation related to COVID-19 and variants thereof and the production of the storm of cytokines which also perpetuates this picture.

Thus, if the ratio between AA and EPA can be normalized the storm of cytokines can be suppressed. Here, the use of EPA-FFA plays a double role - not only to stop the cascade of AA, but also immediately suppresses the activity of NFκB, and as such, reduces the inflammatory storm that is shown in COVID-19 and variants thereof. This inflammatory storm is attached to the development of the stress in the respiratory system of the patients that are dying - regardless of the age of these patient. Thus, an increase in the levels of EPA-FFA can provide a new therapeutic direction for treatment of COVID-19 and variants thereof.

Preferably, the EPA-FFA used for making the medical preparations, medicaments or compositions in accordance with the invention is of at least about 90% purity and will contain no more than minimal or pharmaceutically insignificant amounts of any other polyunsaturated fatty acids. A purity of more than 90% is recommended with the highest commercially available grade (about 99% purity), which is substantially free of any other polyunsaturated fatty acids, being the most preferred material.

Thus, according to one aspect of the present invention, highly purified eicosapentaenoic acid as a free fatty acid is used to make a medical preparation or medicament for the treatment of coronavirus.

EPA may be found in fish oil, plants or microorganisms as free fatty acids or in conjugated forms such as acylglycerols, phospholipids, sulfolipids or glycolipids, and may be extracted through a variety of means well-known in the art. Such means may include extraction with organic solvents, such as methanol and chloroform, sonication, supercritical fluid extraction using for example carbon dioxide, and physical means such as presses, or combinations thereof. Where desirable, the aqueous layer can be acidified to protonate negatively charged moieties and thereby increase partitioning of desired products into the organic layer. After extraction, the organic solvents can be removed by evaporation under a stream of nitrogen. When isolated in conjugated forms, the products may be enzymatically or chemically cleaved to release the free fatty acid or a less complex conjugate of interest and can then be subject to further manipulations to produce a desired end product.

If further purification is necessary, standard methods can be employed. Such methods may include extraction, treatment with urea, fractional crystallization, HPLC, fractional distillation, silica gel chromatography, high speed centrifugation or distillation, or combinations of these techniques. Protection of reactive groups, such as the acid or alkenyl groups, may be done at any step through known techniques, for example alkylation or iodination. Protecting groups may be removed at any step. Desirably, purification of fractions containing EPA may be accomplished by initial esterification, treatment with urea, supercritical fluid extraction and chromatography with the subsequent isolation of the free fatty acid.

In order to isolate EPA from the triglyceride it is necessary to free the fatty acids by hydrolysis or ester exchange in order that purification can be achieved. Purification can be achieved by techniques such as fractional distillation, molecular distillation and chromatography. A particularly desirable chromatographic method employs super-critical fluids using, for example, carbon dioxide as the mobile phase, such as described in European Patent EP 0 712 651. It has been found that using such techniques EPA may be purified to levels approaching 100 per cent. For practical reasons the EPA may be purified as its ethyl or methyl ester and hydrolyzed back to the free fatty acid form. Purification enables a product to be prepared which is highly concentrated and free from other fatty acids that are less desirable in the finished product. In addition, other chemicals entities such as mono- and di-glycerides, hydrocarbons, pesticide residues and the like can be removed. The highly purified EPA is thus suitable for human ingestion as it contains substantially reduced levels of toxins, compounds contributing to unpalatability or undesirable fatty acids such as saturated fatty acids. The free fatty acid form of EPA can be absorbed in the gut easily without need of prior enzymatic conversion. Using this method about 150 kg of impure EPA can be converted into 50 kg of essentially pure EPA-FFA, that being at least has a purity of at least 90%, preferably 95% and more preferably at least 99%.

If further purification is necessary, standard methods can be employed. Such methods may include extraction, treatment with urea, fractional crystallization, HPLC, fractional distillation, silica gel chromatography, high speed centrifugation or distillation, or combinations of these techniques. Protection of reactive groups, such as the acid or alkenyl groups, may be done at any step through known techniques, for example alkylation or iodination. Protecting groups may be removed at any step. Desirably, purification of fractions containing EPA may be accomplished by initial esterification, treatment with urea, supercritical fluid extraction and chromatography with the subsequent isolation of the free fatty acid.

A preferred EPA free fatty acid is commercially available under the tradename ALFA^{™} (S.L.A. Pharma, UK). This PUFA is 99% pure EPA, in a free fatty acid form and formulated into a pH-dependent, enteric-coated capsules designed to ensure release of the contents in the small intestine at pH 5.5. Key advantages of this preparation of EPA are its high degree of purity compared with many fish oil products, its presentation as the free fatty acid maximizing systemic bioavailability, ease of dosage in 500 mg capsules and a delayed-release profile, which minimizes gastro-intestinal side-effects.

The term "treatment" or "treating" as used herein refers to either (1) the prevention of the coronavirus, or (2) the reduction or elimination of symptoms of the coronavirus (therapy).

The terms "prevention", "prevent" or "preventing" as used herein refers to inhibiting, averting or obviating the onset or progression of the virus.

The term "respiratory viruses" as used herein refers to influenza virus, respiratory syncytial virus, parainfluenza viruses, metapneumovirus, rhinovirus, coronaviruses, adenoviruses, and bocaviruses. Such viral infections commonly effect the upper or lower respiratory tract and can include the common cold, bronchiolitis, croup, and pneumonia.

The term "cytokine" as used herein is meant to include any one of the groups of hormone-like mediators produced by T and B lymphocytes. Representative cytokines include but are not limited to Interleukin-1 (IL-1), IL2, IL3, IL4, IL-5, IL-6, IL-7, IL-8, IL-9, IL-10, IL-11, IL-12, IL-13, IL-15, IL-18, Interferon gamma (IFN-.gamma.), Tumor Necrosis Factor alpha (TNF-.alpha.) and Transforming Growth Factor-beta (TGF-.beta.).

The phrase "modulating an immune response" or "modulation of an immune response" as used herein includes downregulation, inhibition or decreasing an immune response as defined herein.

The term "activity", "biological activity" or "functional activity", as used herein refers to an activity exerted by the EPA-FFA, as determined *in vivo,* or *in vitro,* according to standard techniques.

Also, within the scope of this invention is the administration of EPA-FFA prophylactically. Administration of EPA-FFA of the present invention can occur prior to the manifestation of symptoms of the COVID-19 virus and variants thereof, such that the virus is prevented or, alternatively, delayed in its progression. The prophylactic methods of the present invention can be carried out in a similar manner to the therapeutic methods described herein, although dosage and treatment regimens may differ.

This present invention includes treatment or therapy of patients infected with COVID-19 and variants thereof including subjects with symptomatic or asymptomatic COVID-19 infections and variants thereof.

Further, it is considered beneficial to administer the EPA-FFA prophylactically as a preventive method to subjects showing no evidence of the virus.

The EPA-FFA of the present invention is administered in a manner compatible with the dosage formulation, and in such amount as being therapeutically effective. The quantity to be administered depends on the subject to be treated, capacity of the subject's immune system to generate a cellular immune response, and degree of protection desired. Precise amounts of active ingredient required to be administered depend on the judgment of the practitioner and are peculiar to each individual. However, suitable dosage ranges are of the order of about 500 mg to about 4 gram per day.

The term "effective amount" as used herein includes an amount effective, at dosages and for periods of time necessary, to achieve the desired result, e.g., sufficient to modulate the immune response. An effective amount of EPA-FFA, as defined herein may vary according to factors such as the disease state, age, and weight of the subject. Dosage regimens may be adjusted to provide the optimum therapeutic response.

The EPA-FFA compound has at least has a purity of at least 90%, preferably 95% and more preferably at least 99% pure. The EPA-FFA is administered in an amount from about 500 mg to 4 g per day and more preferably from about 250 mg to about 4 g and most preferably from about 1 g to 3 g per day. This dosage may be administered parenterally or orally in one or more doses at various intervals daily, preferably orally once daily. Notably, the tolerability of 99% pure EPA as ALFA^{™} capsules is excellent and the predominant small bowel delivery of EPA minimized any unpleasant taste and smell sensations that have previously hampered therapy with other fish oil preparations. If delivered daily, the administration of EPA-FFA is most beneficial when administered daily for at least a month and more beneficial for up to six months which provides sufficient time to provide a normalize ratio between AA and EPA.

EPA-FFA may change the course of SARS-CoV-2 infections by modulating immune response and protecting patients from its most severe complications. EPA as a free fatty acid is very promptly absorbed and incorporated into the body's phospholipid membranes, and then starts to act very quickly. Once incorporated into cell membranes, EPA-FFA significantly affects the production of pro-inflammatory mediators such as IL-6 and leukotriene B4 that play a crucial role in starting and maintaining the inflammatory process in the lungs. Moreover, EPA-FFA metabolism generates pro-resolving mediators and bioactive metabolites, that enhance innate microbial killing and organ protection.

The EPA-FFA alone or in combination with another therapeutic agent used to treat COVID-19 and variants thereof may be formulated in multiple delivery modes. The active agent can be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

A solid composition form may include a solid carrier and one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredients. In tablets, the active ingredients are mixed with a carrier having the necessary compression properties in suitable proportions and compacted in the shape and size desired. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g., cellulose derivatives, preferably sodium carboxymethyl cellulose solution); alcohols (including monohydric alcohols and polyhydric alcohols, e.g., glycols) and their derivatives, and oils (e.g., fractionated coconut oil and arachis oil). For parenteral administration, the carrier can also be an oily ester such as ethyl oleate and isopropyl myristate. Sterile liquid carriers are useful in sterile liquid form compositions for parenteral administration.

The preferred free fatty acid form of Eicosapentaenoic acid (EPA-FFA) is a n-3 polyunsaturated long chain fatty acid (n-3PUFAs) formulated in gastro-resistant 500 mg capsules. Compared to other n-3PUFAs, EPA-FFA has the fastest pharmacokinetic, leading to a reduced time for its incorporation in the cells, thus allowing EPA-FFA to quickly begin its biological effect that persists for weeks after treatment cessation. (3)

In patients affected by SARS-Cov2 infection, the immune response, which in theory would help fighting the infection, can be exaggerated (cytokine storm), thus damaging the organ tissues even more than the infection itself, causing the need for intensive care, and sometimes resulting in death. (4)

To counteract and inhibit the patient's excessive immune response, immunosuppressive drugs such as anti-Interleukin-6 receptor, tocilizumab, are currently prescribed. (5) However, the immunosuppressive therapies may lower the patient's immune response to such a degree that would result in the increased risk of a rebound SARS-Cov2 infection, and of overlapping, and possibly deadly, opportunistic infections. Markers able to predict those individuals more prone to a cytokine storm are not specific and accurate; thus, it is difficult to select those patients who will benefit from those who could be harmed from the treatment.

EPA-FFA is not an immunosuppressor, but an immunomodulator as shown in Figure 1: it balances the immune response, rather than inhibiting it. Even at high dosages, EPA-FFA carries no-risk of immunosuppression on healthy individuals. The EPA-FFA's action within the context of a patient whose immune system is overactivated (e.g., in autoimmune diseases, or because of SARS-Cov2 infection), is completely different to the effects of a classic immunosuppressive medication. In fact, by increasing the production of anti-inflammatory mediators, EPA-FFA leads to an indirect inhibition of the overactivated immune response. This is achieved through the removal of those stimuli triggering an increased immune response, finally leading to a reduced inflammation. It has been demonstrated that the competition between Arachidonic Acid and EPA-FFA for cyclooxygenases and lipoxygenases increases, for example, the production of leukotriene B-5 (with poor biological activity) at the expenses of leukotriene B-4 (with a potent pro-inflammatory activity), and cytokines such as IL-10, known for its potent anti-inflammatory effect. Finally, administration of EPA-FFA leads to the production of small protective molecules (resolvins, defensins and maresins), known to have powerful anti-inflammatory and damaged-tissues repair effects. (6)

Based on this rationale, EPA-FFA has been recently cleared by the Medicines and Healthcare products Regulatory Agency for a phase II clinical trial study to treat hospitalized patients with SARS-Cov2 infection, as discussed below. (7)

The clinical study includes evaluation of EPA-FFA efficacy compared to placebo during the 28-day treatment period. Treatment failure is defined as additional or alternative treatment required, or intubation and invasive ventilation, or transfer to intensive care unit, or death.

### EPA-FFA to Treat Hospitalized Patients With COVID-19 (SARS-CoV-2)

A Randomized, Double-blind, Placebo Controlled Study of Eicosapentaenoic Acid (EPA-FFA) Gastro-resistant Capsules to Treat Hospitalized Subjects With Confirmed SARS-CoV-2. This is a double-blind, randomized, placebo controlled phase III study in hospitalized subjects with confirmed SARS-CoV-2. Eicosapentaenoic acid free fatty acid (EPA-FFA) 500 mg gastro-resistant capsules 2g daily (two capsules twice daily). One capsule of EPA-FFA gastro-resistant capsules contains 500 mg EPA-FFA in a capsule containing gelatin, glycerol, sorbitol, titanium dioxide, FD&C blue No. 1, hypromellose phthalate, dibutyl sebacate (EPAspire). The placebo capsules are not visually differentiated from the active treatment.

### Recruitment:

Subjects hospitalized or attended to the hospital with a confirmed diagnosis of SARS-CoV-2, are contacted.
Potential subjects have the opportunity to ask any questions to the researchers.
A member of the research team provides a copy of the information sheet to the subject, who have the opportunity to ask any questions to the researchers.
Subjects expressing an interest in participating are interviewed to explain the study in detail, and discuss the risks, benefits, goals and limitations of the study.

The subject must satisfy the following criteria for entry into the study:
The subject must satisfy the following criteria for entry into the study:
1. Male or female, aged 18 years and above.
2. Provide informed consent prior to any study specific procedure being conducted; for older patients who lack mental or physical capacity, next of kin or legal guardians will be allowed to provide consent on their behalf. This consent can be obtained remotely by telephone to the next of kin, or by a doctor with relevant experience in COVID-19 disease not directly involved in the study acting as the patient's advocate and then subsequently informing the next of kin (e.g., by a telephone call also offering them an opportunity to review and agree the ICF with them; the patient may then continue in the study or withdraw at a later date if the next of kin subsequently decides to withdraw consent).
3. Positive local approved test to confirm diagnosis of SARS-CoV-2 within 7 days prior to baseline.
4. Classified as moderate or severe based on the modified WHO/NIH baseline severity criteria. Moderate: evidence of lower respiratory disease by clinical assessment (e.g., signs or symptoms of lung infection) or by chest X-ray/CT/ultrasound imaging (e.g., viral pneumonia, lung infiltrates) and a saturation of oxygen (SaO₂) ≥ 94% on room air at sea level. Severe: respiratory frequency >30 bpm, SaO₂ < 94% on room air at sea level, ratio of arterial partial pressure of oxygen to fraction of inspired oxygen (PaO₂/FiO₂) < 300 mmHg, or lung infiltrates >50%.
5. Hospitalized or attended the hospital due to clinical and/or virological diagnosis of SARS-CoV-2; subsequent follow up after screening may be carried out in hospital (hospitalized) or at a COVID-19 hospital OP clinic as clinically indicated at the investigator's discretion. Where it is not possible for the subject to attend a hospital OP clinic, then providing a suitably trained healthcare professional (e.g., part of the clinical research team) as directed by the investigator, is available to visit the subject at home to conduct the necessary clinical and SaO₂ assessments and blood tests, subsequent assessments post-hospitalization or ED visit may be conducted at the subject's home.

### Other criteria may include;

6. Evidence of viral pneumonia (either CT scan or Chest Ultrasonogram).
7. Fever defined as temperature ≥ 36.6°C armpit, ≥ 37.2°C oral, ≥ 37.8°C rectal.

### Exclusion criteria:

The subject will be excluded from the study if any of the following applies:
1. Subjects intubated prior to administration of IMP.
2. Subjects requiring mechanical ventilation at screening.
3. Subjects who are unable to swallow capsules easily.
4. Known allergic reaction or intolerant to fish or fish oils.
5. Known allergic reaction to excipients of IMP.
6. Subjects who are pregnant or breast-feeding at screening.
7. Subjects who are taking other fish-oil supplements (e.g. cod liver oil) who are unwilling to stop them for the duration of the study.
8. Subjects taking warfarin or other anti-coagulants.
9. Subjects who have used another investigational drug in the past 48 hours or 5 half-lives, whichever is longer, prior to Screening.
10. Subjects who are participating in other clinical studies at the same time.
11. Evidence of multi-organ failure.
12. Subjects with a do not resuscitate code.
13. Subject is deemed, by the investigator, unlikely to be able to comply with the requirements of the protocol.
14. Subject is deemed, by the investigator, likely to require transfer to the intensive care unit (ICU) or unlikely to survive for at least 48 hours.
15. Subjects with any laboratory tests from samples taken at screening considered clinically significant.

Screening Procedures. Potentially eligible subjects provide informed consent prior to any study specific procedures being conducted. Following the provision of informed consent, the subject's demographics and medical history especially that relating to SARS-CoV-2 is documented.
- A physical examination and checks on vital signs (BP, pulse, temperature, respiration) is be conducted. Female subjects of child-bearing potential undergo a urine pregnancy test. A blood sample is drawn for routine hematology and biochemistry. Subjects also undergo tests for oxygen saturation, PaO2/FiO2 and interleukin-6 (IL-6).
- Subjects are asked to provide details of any concomitant medications. Subjects with confirmed diagnosis of SARS-CoV-2, compliance with the inclusion and exclusion criteria and providing informed consent are registered on the e-CRF to obtain a randomization number.
- Baseline/Randomization A physical examination and checks on vital signs (BP, pulse, temperature, respiration) is conducted. Subjects undergo tests for oxygen saturation, PaO₂/FiO₂ and interleukin-6. Hematology and biochemistry test (from screening) results are confirmed as being acceptable.

The study center dispenses the EPA-FFA under blinded conditions according to a permuted block randomization sequence (1: 1 ratio for the two subject groups).

The subjects are trained on the dosing and asked to administer two capsules (500 mg of EPA-FFA) twice daily for 4 weeks. Subjects are then be provided with the EPA-FFA capsules on a daily basis by a suitably qualified and delegated member of the Investigator's team, for the duration of the treatment phase.

Treatment Phase (Week 1-3) All subjects receive standard of care treatment throughout the treatment phase on a day to day basis. This includes assessment of additional or alternative medication required for the treatment of SARS-CoV-2, requirement for intubation and invasive ventilation, requirement to transfer to intensive care unit or death. On a weekly basis, a physical examination and checks on vital signs (BP, pulse, temperature, respiration) are conducted. Subjects undergo tests for oxygen saturation, PaO2/FiO2 and IL-6. Subjects are asked to provide details of any concomitant medications and changes in condition via adverse event (AE) query.

Week 4 A physical examination and checks on vital signs (BP, pulse, temperature, respiration) is conducted. Subjects undergo tests for oxygen saturation, PaO2/FiO2 and IL-6. Female subjects of child-bearing potential undergo a urine pregnancy test. A blood sample is drawn for routine hematology and biochemistry. Subjects are asked to provide details of any concomitant medications and changes in condition via AE query.

Week 6 (Follow -up) 6 weeks after randomization, the subjects are contacted to check the occurrence of any other adverse events and review of medications. Hematology and biochemistry test (from week 4) results are confirmed as being acceptable.

### Efficacy of EPA-FFA:

Time to treatment failure during the 28-day treatment period. Treatment failure is defined as additional or alternative treatment required, or intubation and invasive ventilation, or transfer to intensive care unit, or death.

Time to and amount of clinical improvement during the 28-day treatment period. To determine whether EPA-FFA gastro-resistant capsules decreases the time to and amount of clinical improvement as determined by the WHO 9-point ordinal scale during the study.
- Change in recovery and survival rate. To determine whether EPA-FFA gastro-resistant capsules increases the number of subjects alive and discharged home without supplemental oxygen therapy.
- Reduction of CRP, D-Dimers and IL-6. To determine whether EPA-FFA gastro-resistant capsules decreases CRP, IL-6 and D-dimers during the study.
- Increase in IFN-γ. To determine whether EPA-FFA gastro-resistant capsules increases IFN-γ during the study.
- Reduction in proinflammatory chemokines and cytokines. To determine whether EPA-FFA gastro-resistant capsules decreases other proinflammatory chemokines and cytokines.
- Increase in oxygen saturation. Time to and number of days with oxygen saturation >95% without oxygen inhalation during the 28-day treatment period.
- PaO₂/FiO₂ >300 mmHg increase. Time to and number of days with PaO₂/FiO₂ >300 mmHg without oxygen inhalation during the 28-day treatment period.
- Reduction of IL-6. Change in IL-6 level during the 28-day treatment period.
- Mortality rate reduction. To determine whether EPA-FFA gastro-resistant capsules decreases mortality rate during the study.
- Reduction in ICU stays. To determine whether EPA-FFA gastro-resistant capsules decreases ICU days during the study.
- Reducing hospitalization days. To determine whether EPA-FFA gastro-resistant capsules decreases hospitalization days during the study.
- Reduction in need for mechanical ventilation. To determine whether EPA-FFA gastro-resistant capsules decreases the need for invasive mechanical ventilation during the study
- Fever reduction. Time to and number of days with fever normalization < 36.6°C armpit, < 37.2°C oral, < 37.8°C rectal, during the 28-day treatment period

### References

The contents of the references cited herein is incorporated by reference herein for all purposes.
1. Pan, Y., Guan, H., Zhou, S. et al. Initial CT findings and temporal changes in patients with the novel coronavirus pneumonia (2019-nCoV): a study of 63 patients in Wuhan, China. Eur Radiol 30, 3306-3309 (2020).
2. Ando N, Hara M, Shiga K, Yanagita T, Takasu K, Nakai N, Maeda Y, Hirokawa T, Takahashi H, Ishiguro H, Matsuo Y, Takiguchi S. Eicosapentaenoic acid suppresses angiogenesis via reducing secretion of IL-6 and VEGF from colon cancer-associated fibroblasts. Oncol Rep. 2019 Jul;42(1):339-349. doi: 10.3892/or.2019.7141. Epub 2019 May 2. PMID: 31059084.
3. Scaioli E, Cardamone C, Liverani E, Munarini A, Hull MA, Belluzzi A. The Pharmacokinetic Profile of a New Gastroresistant Capsule Preparation of Eicosapentaenoic Acid as the Free Fatty Acid. Biomed Res Int 2015; 2015: 360825.
4. Zhou F, Yu T, Du R, et al. Clinical course and risk factors for mortality of adult inpatients with COVID-19 in Wuhan, China: a retrospective cohort study. Lancet 2020. DOI:10.1016/S0140-6736(20)30566-3.
5. Luo P, Liu Y, Qiu L, Liu X, Liu D, Li J. Tocilizumab treatment in COVID-19: a single center experience. J Med Virol 2020; published online April 6. OI:10.1002/jmv.25801.
6. Scaioli E, Liverani E, Belluzzi A. The Imbalance between n-6/n-3 Polyunsaturated Fatty Acids and Inflammatory Bowel Disease: A Comprehensive Review and Future Therapeutic Perspectives. Int J Mol Sci 2017; 18. DOI:10.3390/ijms18122619.
7. EPA-FFA to Treat Hospitalised Patients With COVID-19 (SARS-CoV-2) - Full Text View - ClinicalTrials.gov. https://clinicaltrials.gov/ct2/show/NCT04335032 (accessed April 13, 2020).

## Claims

1. A method for treating and reducing the effects of COVID-19 and variants thereof in a subject, the method comprising administering to the subject a therapeutic amount of eicosapentaenoic acid in the free fatty acid (EPA-FFA) form having purity of at least 95%.

2. The method of claim 1, wherein the purity of EPA-FFA is at least 99%.

3. The method of claim 1, wherein the therapeutic amount is in an amount 500 mg to about 4 g per day.

4. The method of claim 3, wherein the amount is from about 1 g to 3 g per day.

5. The method of claim 4, wherein the amount is delivered for a period of one month to six months to normalize the AA:EPA ratio between 1: 1 to about 5:1.

6. A method for alleviating the cytopathic destructive effects of COVID-19 and variants thereof in a human patient infected with such a virus comprising administering to said patient a therapeutically-effective amount of EPA-FFA having a purity of at least has a purity of at least 90%, preferably 95% and more preferably at least 99% or a composition containing such a compound.

7. A method to modulate an immune response caused by increase in the expression of interleukins and the release of arachidonic acid (AA), the method comprising the administration to a subject a therapeutically effective amount of EPA-FFA having a purity of 99% to reduce the level of interleukins and release of AA.

8. A method to reduce a late complication event of COVID 19 and variants thereof, wherein the late complication is lung fibrosis in a subject, wherein EPA-FFA is administered in an amount to reduce IL-6 and VEGF production and the secondary neo angiogenesis that ultimately generates pulmonary fibrosis.

9. Use of EPA-FFA as a medicament for the treatment of COVID-19 and variants thereof.

10. Use of EPA-FFA in the manufacture of a medicament for the treatment of COVID-19 and variants thereof.
